# EUROPÄISCHE PATENTSCHRIFT

(11) **EP 1 132 432 B1**
(45) Veröffentlichungstag und Bekanntmachung des Hinweises auf die Patenterteilung: **08.02.2006**
(21) Anmeldenummer: 01810223.6
(22) Anmeldetag: 05.03.2001
(51) Int. Cl.: C09B 19/02

(54) **Verfahren zur Herstellung von Pigmenten**
Preparation process of pigments
Procédé de préparation de pigments

(30) Priorität: 06.03.2000 CH 4302000
(43) Veröffentlichungstag der Anmeldung: 12.09.2001
(73) Patentinhaber: Clariant Finance (BVI) Limited, Road Town, Tortola (VG)
(72) Erfinder: Kaul, Bansi Lai, 4105 Biel Benken (CH); Piastra, Bruno, 68330 Huningue (FR); Steffanut, Pascal, 68000 Colmar (FR)
(74) Vertreter: Hütter, Klaus

(56) Entgegenhaltungen:
- EP-A- 0 889 046
- EP-A- 0 911 337
- EP-A- 0 945 455
- DE-A- 10 052 858
- GB-A- 2 228 738
- GB-A- 2 284 427

## Beschreibung

Gegenstand der Erfindung ist ein besonders vorteilhaftes Verfahren zur Herstellung von Triphendioxazin-Verbindungen und besonders vorteilhaften Zwischenprodukten.

EP 0 199 056 A2 beschreibt ein Verfahren zur Herstellung von Triphendioxazinen durch oxidativen Ringschluss von spezifischen, p-Amino-substituierten 2,5-Diarylamino-1,4-benzochinonen, bestehend darin, dass man in wasserhaltiger Schwefelsäure in Gegenwart von Mangandioxid bei Temperaturen von -10 bis +25°C arbeitet.

Die Europäische Patentanmeldung EP 0889046 A1 beschreibt symmetrisch disubstituierte Triphendioxazin-Verbindungen der folgenden allgemeinen Formel (1) 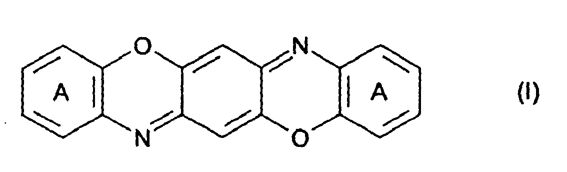
in der die mit A bezeichneten Kerne u.a. linear in 2,3- und 9,10-Stellung anellierte Ringe mit Restgliedern u.a. der Formeln -NR₁₋(CO)ₘ-NH- und -NR₁₋(CO)ₘ-O- tragen; wobei R₁ Wasserstoff, C₁₋₄Alkyl oder Phenyl, vorzugsweise Wasserstoff, Methyl oder Ethyl; und m 1 oder 2 bedeuten.

Das in EP 0889046 A1 offenbarte Herstellungsverfahren geht von Vorprodukten, d.h. Aminoverbindungen aus, die in ortho-Stellung durch eine Alkoxygruppe substituiert sind und nur durch eine relativ aufwendige Synthese erhältlich sind. Das beschriebene Verfahren ist gekennzeichnet durch die Verwendung von 2,3,5,6-Tetrachloro-1,4-benzochinon und führt zu am zentralen Ring chlorierten Zwischenverbindungen. Die Ringschlussreaktion erfolgt unter Dechlorierung zu den chlorfreien Triphendioxazinverbindungen der allgemeinen Formel (1). Die Kondensationsreaktion führt im allgemeinen nicht vollständig zu den chlorfreien Verbindungen der Formel (I); es entstehen daneben praktisch immer eine gewisse Menge Monochlor- und Dichlorverbindungen.

Die Aufgabe der Erfindung besteht in der Bereitstellung eines Verfahrens, das von einfach zugänglichen am zentralen Ring nicht chlorierten Vorprodukten ausgeht und über nichtchlorierte Zwischenverbindungen zu den gewünschten am zentralen Ring vollständig chlorfreien Triphendioxazinverbindungen führt.

Überraschenderweise wurde festgestellt, dass auch Aminoverbindungen, die in ortho-Stellung nicht substituiert sind, als Vorprodukte geeignet sind, sofern die der Umsetzung mit einem Benzochinon folgende Cyclisierung erfindungsgemäss mit Braunstein (Mangandioxid) und 80 bis 100%iger, vorzugsweise 90 bis 95%iger Schwefelsäure erfolgt. Unerwartet ist auch, dass bei der Cyclisierung keine Sulfonierung am zentralen Ring erfolgt, sondern direkt die Triphendioxazinverbindungen der allgemeinen Formel (I) erreicht werden.

Nach der Umsetzung von 1 Mol einer Verbindung der allgemeinen Formel (II) 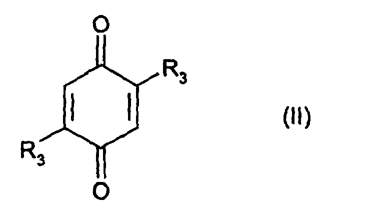 worin R₃ Wasserstoff, einen C₁₋₁₀Alkoxyrest, Halogen oder Hydroxy bedeutet, mit 2 Mol einer Verbindung der allgemeinen Formeln (III) (= Vorprodukte) 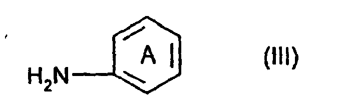
worin der mit A bezeichnete Kern linear in 3,4-Stellung anelliert einen Ring mit den Restgliedern der Formeln -NR₁(CO)ₘ-NR₂ oder -O-CO-NR₂ enthält; m 1 oder 2 ist; und R₁ bzw. R₂ unabhängig voneinander, Wasserstoff, einen C₁₋₈Alkylrest, einen substituierten oder unsubstituierten Phenyl-, Benzyl-, Naphthyl- oder Benzanilid-Rest, einen substituierten oder unsubstituierten C₅₋₆Cycloalkyl-Rest oder einen Rest der Formel 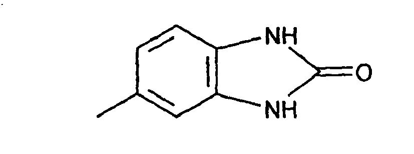 bedeuten, folgt als kennzeichnender Verfahrensschritt die mit Braunstein (MnO₂) und 80 bis 100%iger, vorzugsweise 90 bis 95%iger Schwefelsäure durchgeführte Cyclisierung.

Bevorzugte Verbindungen der allgemeinen Formel (III) sind 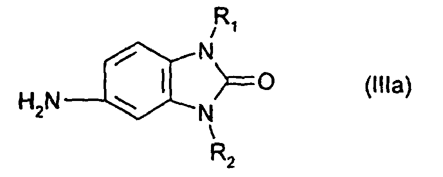 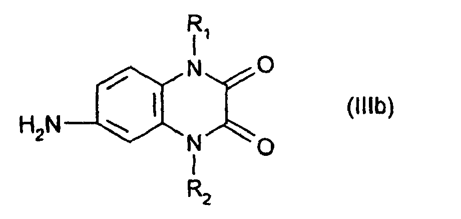 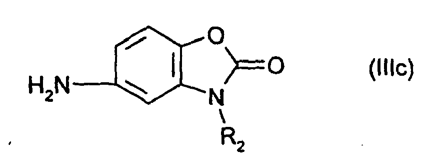 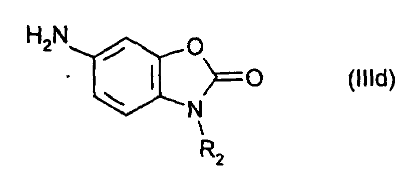
wobei R₁ und R₂ wie oben definiert sind.

Die Kondensationsreaktion der Verbindung (II) mit der Verbindung (III) resp. (IIIa-d) wird in einem Lösungsmittel in Gegenwart einer anorganischen oder organischen Säure als Katalysator zwischen 50 und 160°C durchgeführt. Sie kann auch in Wasser oder Eisessig durchgeführt werden. Die Kondensationsreaktion führt zu den Zwischenprodukten der allgemeinen Formel (IV), worin die mit A bezeichneten Kerne wie oben für Formel (III) definiert sind.

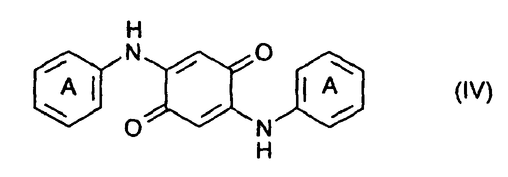

Der Vorteil dieses Verfahrens liegt darin, dass von den Zwischenprodukten (III), resp. (IIIa), (IIIb), (IIIc) und (IIId) ausgegangen werden kann, die im Gegensatz zu den Aminoverbindungen, die in ortho-Stellung eine Alkoxygruppe tragen, relativ einfach zugänglich sind. Als Ausgangsverbindung (II) wird vorzugsweise das 2,5-Dihydroxy-1,4-benzochinon eingesetzt.

Bevorzugte Lösungsmittel der Kondensationsreaktion sind hochsiedende Lösungsmittel wie zum Beispiel N-methylpyrrolidon, Dimethylformamid oder ortho-Dichlorbenzol.

Die Herstellung der Vorprodukte (IIIa), (IIIb), (IIIc) und (IIId) ist in der EP 0911337 A1 beschrieben.

Vorzugsweise bedeuten R₁ und R₂ unabhängig voneinander, Wasserstoff, einen Methyl-Rest, einen Ethyl-Rest, einen (n,i) Propyl-Rest, einen (n, i, sek., tert.) Butyl-Rest, einen Cyclohexyl-Rest, einen substituierten oder unsubstituierten Benzanilid-Rest, einen Naphthyl-Rest, einen Rest der Formel 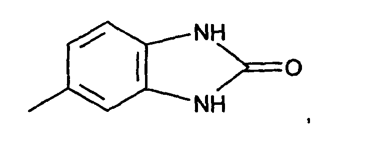 einen unsubstituierten Phenyl-Rest, einen Phenyl-Rest, der einfach oder mehrfach substituiert ist durch Reste aus der Gruppe Halogen, vorzugsweise Chlor, Nitro, Phenyl, C₁₋₈₋Alkyl, vorzugsweise C₁₋₄-Alkyl, und C₁₋₂-Alkoxy.

Der obengenannte substituierte Phenylrest in der Definition von R₁ und R₂ ist vorzugsweise ein Rest der Formeln (a) bis (q)

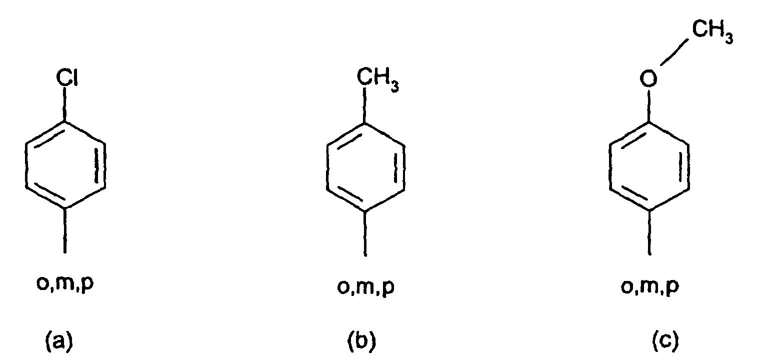

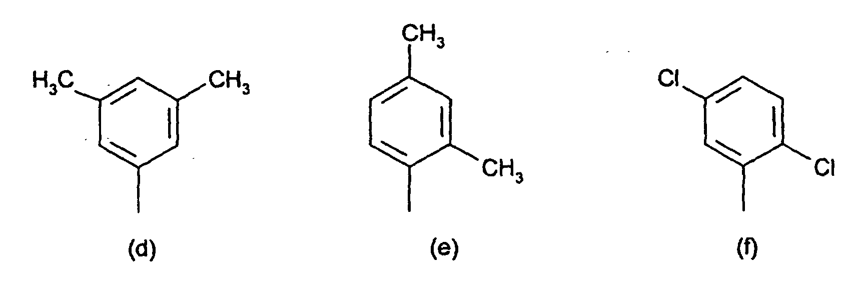

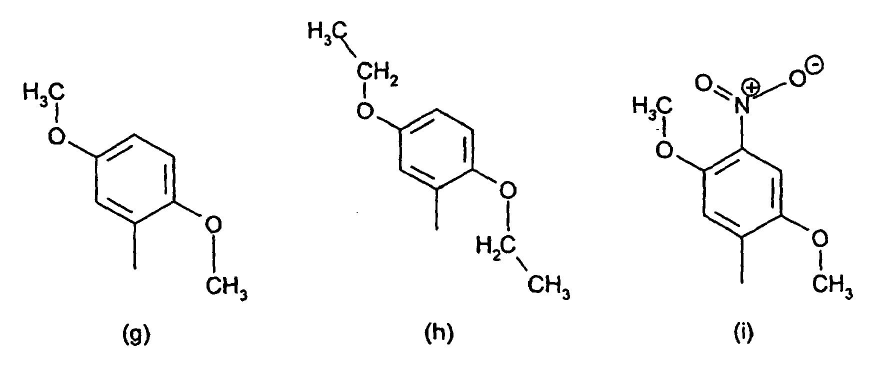

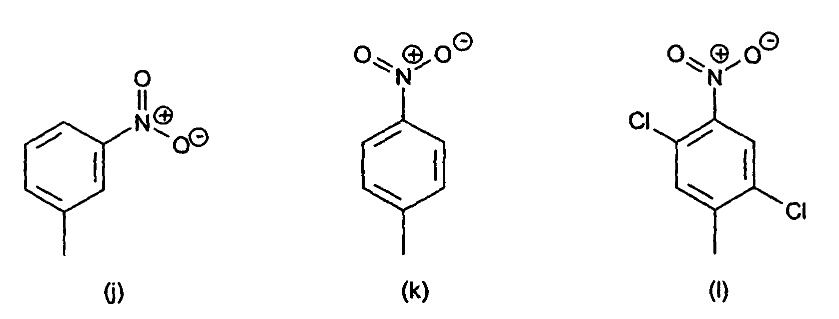

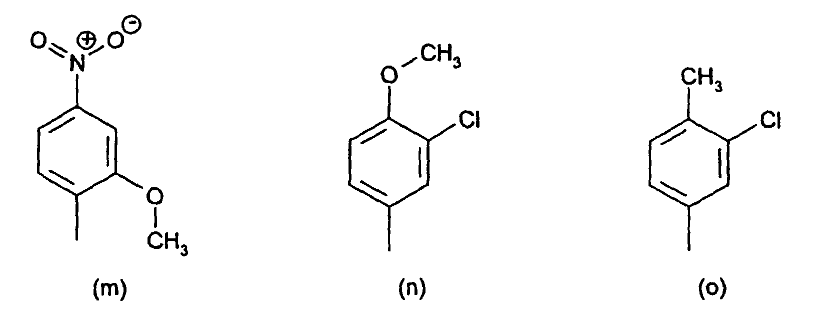

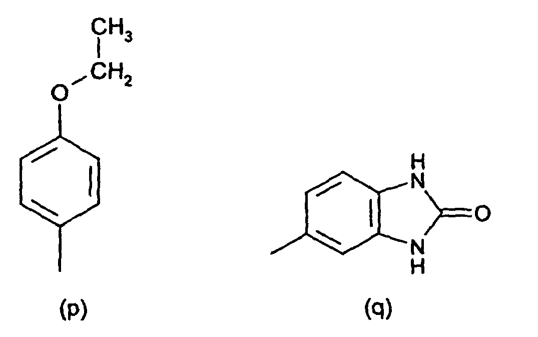

Der obengenannte substituierte Benzanilid-Rest in der Definition von R₁ und R₂ ist vorzugsweise ein Rest der Formeln (r) und (s)

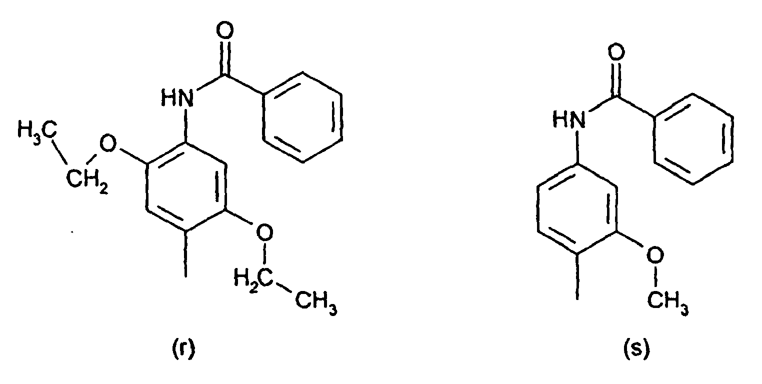

Besonders bevorzugte Verbindungen der Formel (III) sind Verbindungen, die den folgenden Formeln (1) bis (18) entsprechen

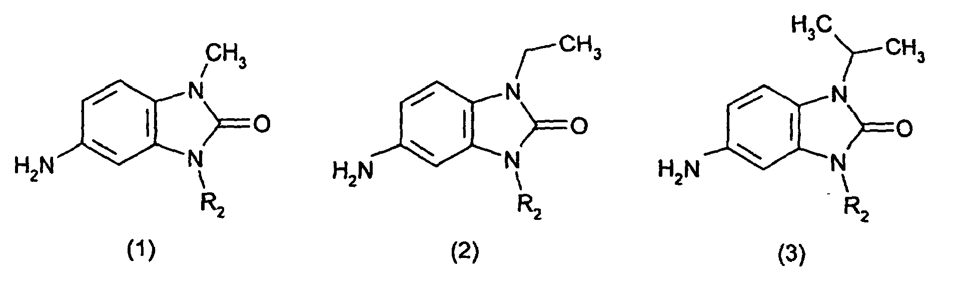

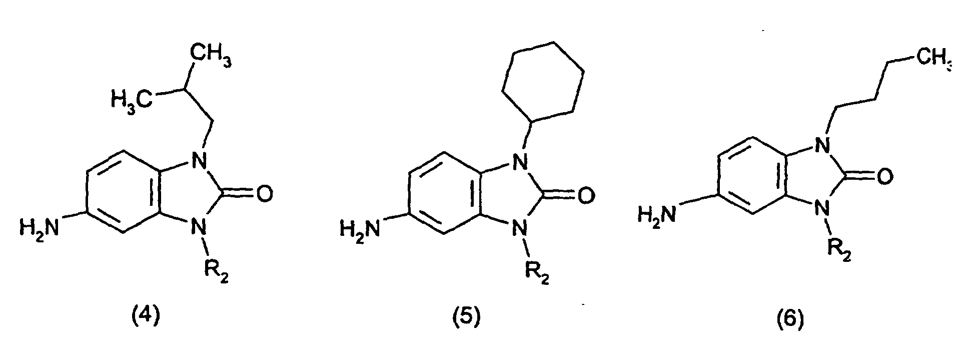

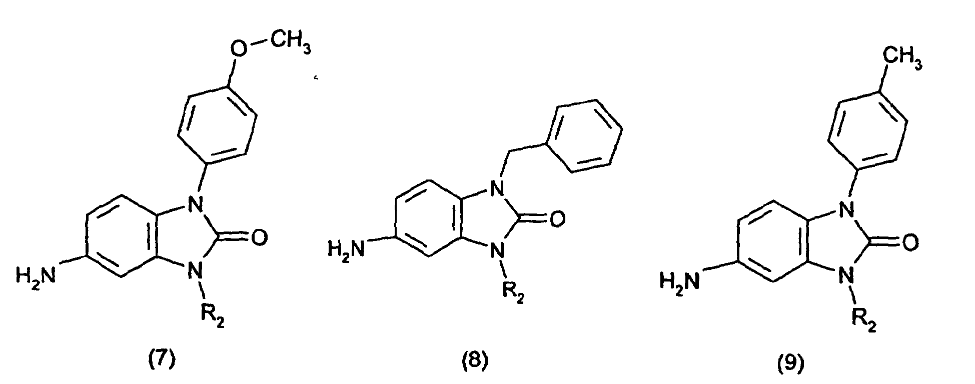

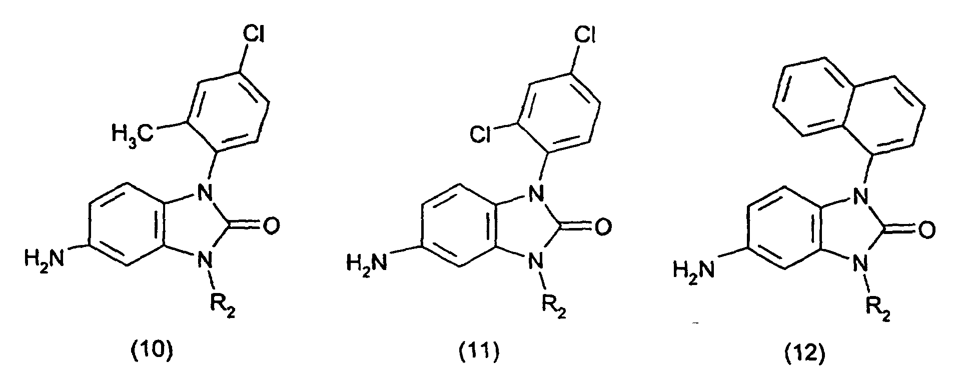

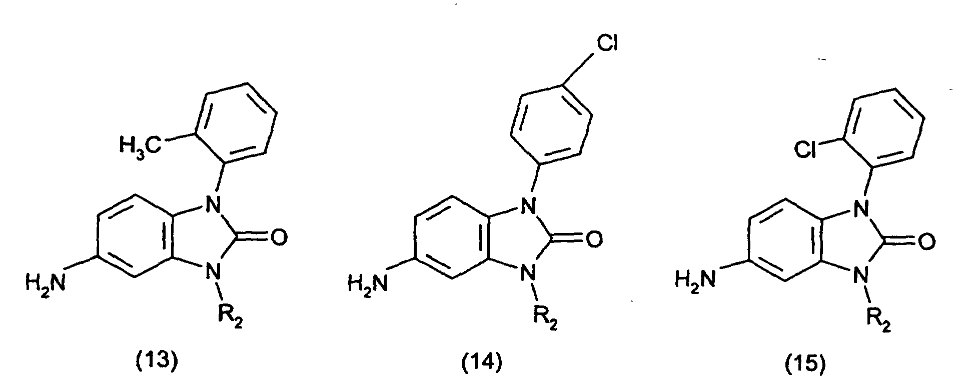

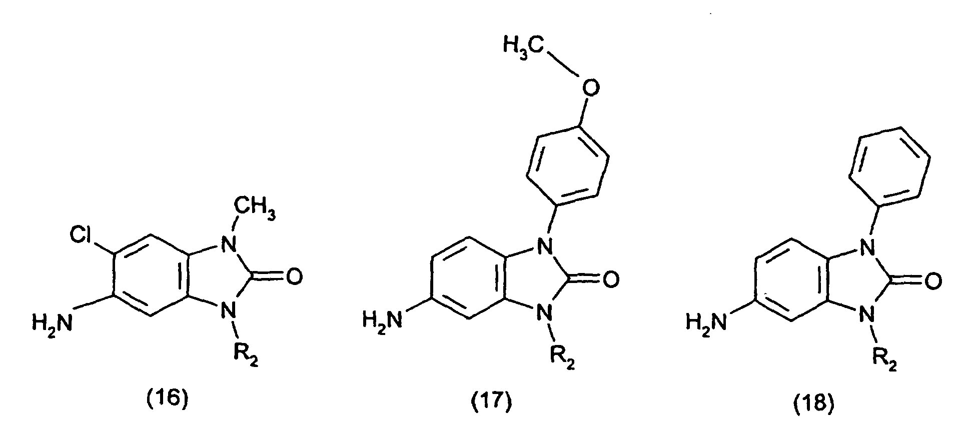

Nach dem erfindungsgemässen Verfahren hergestellte Triphendioxazin-Verbindungen der Formel (I) werden als Pigmente verwendet.

Die nach dem erfindungsgemässen Verfahren hergestellten Pigmente eignen sich ausgezeichnet zum Färben von Kunststoffmassen, worunter lösungsmittelfreie und lösungsmittelhaltige Massen aus Kunststoffen oder Kunstharzen verstanden werden (in Anstrichfarben auf öliger oder wässriger Grundlage, in Lacken verschiedener Art, zum Spinnfärben von Viscose oder Celluloseacetat, zum Pigmentieren von Kunststoffen, beispielsweise Polyamid, Polyäthylen, Polystyrol, Polyvinylchlorid, Kautschuk und Kunstleder). Sie können auch in Druckfarben für das graphische Gewerbe, für die Papiermassenfärbung, für die Beschichtung von Textilien oder für den Pigmentdruck Verwendung finden.

Sie können auch in der Kosmetik Verwendung finden.

Die erhaltenen Färbungen zeichnen sich durch ihre hervorragende Hitze-, Licht- und Wetterechtheit, Chemikalienbeständigkeit, ihre Farbstärke und die sehr guten applikatorischen Eigenschaften, z.B. Kristallisierechtheit und Dispergierechtheit und insbesondere durch ihre Migrier-, Ausblüh-, Überlackier- und Lösungsmittelechtheit aus.

Desweiteren sind die nach dem erfindungsgemässen Verfahren hergestellten Pigmente auch geeignet als Farbmittel in elektrophotographischen Tonern und Entwicklern, wie z.B. Ein- oder Zweikomponentenpulvertonern (auch Ein- oder Zweikomponenten-Entwickler genannt), Magnettonem, Flüssigtonern, Polymerisationstonern sowie weitere Spezialtonem (Lit: L.B. Schein, "Electrophotography and Development Physics"; Springer Series in Electrophysics 14, Springer Verlag, 2^{nd} edition, 1992).

Typische Tonerbindemittel sind Polymerisations-, Polyadditions- und Polykondensationsharze, wie Styrol-, Styrolacrylat-, Styrolbutadien-, Acrylat-, Polyester-. Phenol-Epoxidharze, Polysulfone, Polyurethane, einzelne oder in Kombination, sowie Polyethylen und Polypropylen, die noch weitere Inhaltsstoffe, wie Ladungssteuermittel, Wachse oder Fliesshilfsmittel, enthalten können oder im Nachhinein zugesetzt bekommen können.

Ein weiteres Anwendungsgebiet der nach dem erfindungsgemässen Verfahren hergestellten Pigmente ist ihre Verwendung als Farbmittel in Pulver und Pulverlacken, insbesondere triboelektrisch oder elektrokinetisch versprühten Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen (J.F.

Hughes, "Electrostatics Powder Coating", Research Studies Press, John Wiley & Sons, 1984).

Als Pulverlackharze werden typischerweise Epoxidharze, carboxyl- und hydroxygruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Ausserdem sind die nach dem efindungsgemässen Verfahren hergestellten Pigmente geeignet, als Farbmittel in Ink-Jet Tinten auf wässriger und nichtwässriger Basis, sowie in solchen Tinten, die nach dem Hot-melt Verfahren arbeiten.

In den folgenden Beispielen bedeuten die Teile Gewichtsteile und die Prozente Gewichtsprozente. Die Temperaturen sind in Celsiusgraden angegeben. Ein Volumenteil entspricht dem Volumen eines Gewichtsteils Wasser.

### BEISPIEL 1

### 3,11-di-p-tolyl Diimidazo(4,5-b:4',5'-m) triphendioxazin-2,10-dion

In 500 Vol. Teile Eisessig werden 120 Teile 1-(*p*-tolyl)-1,3-Dihydro-5-amino-benzimidazol-2-on und 35 Teile 2,5-Dihydroxy-1,4-benzochinon eingetragen. Das Gemisch wird unter Rühren auf 110°C erhitzt, wird abgekühlt und das auskristallisierte Zwischenprodukt abfiltriert. Dieses wird mit 250 Teilen Eisessig, dann mit 500 Teilen Wasser gewaschen und getrocknet.

Microanalysis : calc. C 70.09 H 4.5 N 14.42 O 10.98%; found C 69.9 H 4.4 N 14.4 O 11.1%.

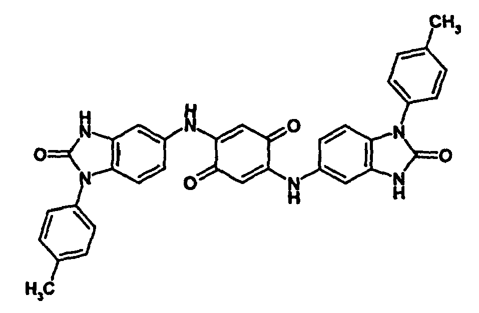

81 Teile dieses Zwischenproduktes werden unter Eiskühlung in 400 Teile H₂SO₄ (90%) eingetragen. Anschliessend werden innert 3 Stunden 29,6 Teile MnO₂ (88%) zugegeben und innert 24 Stunden auf Raumtemperatur erwärmt. Durch Zugabe von 63 Teilen Wasser wird das Kondensationsgemisch auf 80% verdünnt und unter Kühlung mit 5,1 Teilen H₂O₂ versetzt. Das erhaltene Pigment wird abfiltriert und mit 300 Teilen 80% H₂SO₄, danach mit 300 Teilen 50% H₂SO₄ und mit 4000 Teilen Wasser säurefrei gewaschen. Der feuchte Presskuchen wird dann in 500 Teilen Dimethylacetamid suspendiert und das verbleibende Wasser bei 100-150°C abdestilliert. Nach Kühlung auf 120°C wird das Pigment filtriert, dann mit 800 Teilen 120°C heissem Dimethylacetamid und danach mit 400 Teilen Wasser gewaschen und bei 80°C im Vakuum getrocknet. Es werden 60 Teile eines Pigments der obenstehenden Formel erhalten, das Kunststoffe in rotvioletten Tönen mit ausgezeichneter Migrierechtheit färbt. 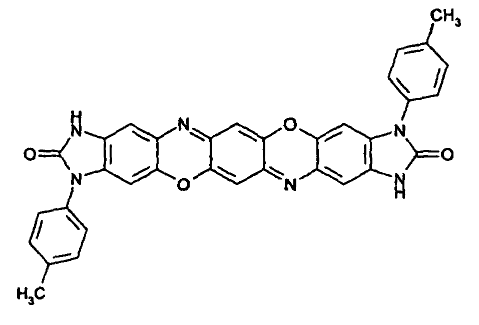
NMR ¹H (360 MHz, D6-DMSO): 2.4 (s, 6H, CH3), 6.3 (s, 2H, aromatic CH), 6.5 (s, 2H, aromatic CH), 6.7 (s, 2H, aromatic CH), 7.3 (dd, 8H, tolyl CH).
Microanalysis: calc. C 70.58 H 3.83 N 14.52%; found C 69.2 H 4.0 N 14.6 %.

### BEISPIEL 2

### 3,11-Dimethyl-diimidazo(4,5-b:4',5'-m)triphendioxazin-2,10-dion

In 400 Vol. Teile Eisessig werden 75 Teile 1-Methyl-1,3-dihydro-5-aminobenzimidazol-2-on, 35 Teile 2,5-Dihydroxy-1,4-benzochinon eingetragen. Das Gemisch wird unter Rühren zum Sieden erhitzt, abgekühlt und das auskristallisierte Zwischenprodukt abfiltriert. Dieses wird mit 200 Teilen Eisessig, dann mit Wasser gewaschen und getrocknet.

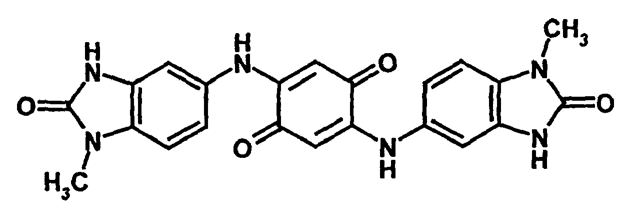

50 Teile dieses Zwischenproduktes werden unter Eiskühlung in 500 Teile H₂SO₄ (95%) eingetragen. Anschliessend werden innert 3 Stunden 13 Teile MnO₂ zugegeben und innert 24 Stunden auf Raumtemperatur erwärmt. Durch Zugabe von 94 Teilen Wasser wird das Kondensationsgemisch auf 80% verdünnt und unter Kühlung mit 3,6 Teilen H₂O₂ versetzt. Das erhaltene Pigment wird abfiltriert und mit 600 Teilen 80% H₂SO₄, danach mit 300 Teilen 50% H₂SO₄ und mit 4000 Teilen Wasser säurefrei gewaschen. Der feuchte Presskuchen wird dann in 500 Teilen Dimethylacetamid suspendiert und das verbleibende Wasser bei 100-150°C abdestilliert. Nach Kühlung auf 120°C wird das Pigment filtriert, dann mit 800 Teilen 120°C heissem Dimethylacetamid und danach mit 400 Teilen Wasser gewaschen und bei 80°C im Vakuum getrocknet.

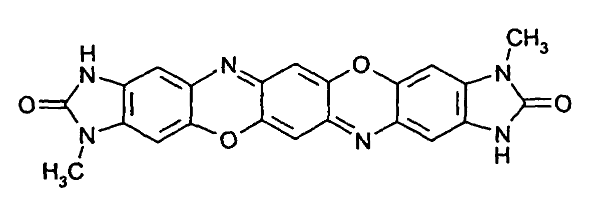

Das erhaltene Pigment der obenstehenden Formel färbt Kunststoffe in rotvioletten Tönen mit ausgezeichneter Migrier- und Lösungsmittelechtheit.

### BEISPIEL 3

### 3,11-Diphenyl-diimidazo(4,5-b:4',5'-m)triphendioxazin-2,10-dion

In 400 Vol. Teile Eisessig werden 128 Teile 1-(phenyl)-1,3-Dihydro-5-amino-benzimidazol-2-on und 35 Teile 2,5-Dihydroxy-1,4-benzochinon eingetragen. Das Gemisch wird unter Rühren auf 110°C erhitzt, abgekühlt und das auskristallisierte Zwischenprodukt abfiltriert. Dieses wird mit 300 Teilen Eisessig, dann mit 500 Teilen Wasser gewaschen und getrocknet.
NMR ¹H (360 MHz, D₆-DMSO): 5.8 (s, 2H, CH quinone), 7.15 (s, 4H, aromatic CH), 7.25 (s, 2H, aromatic CH), 7.5 (m, 2H, aromatic CH), 7.7 (m, 8H, aromatic CH), 9.3 (s, 2H, NH), 11.2 (s, 2H, NH).
Microanalysis: calc. C 69.3 H 4.0 N 15.15 O 11.54 %; found C 68.4 H 3.9 N 15.5 O 11.7 %. 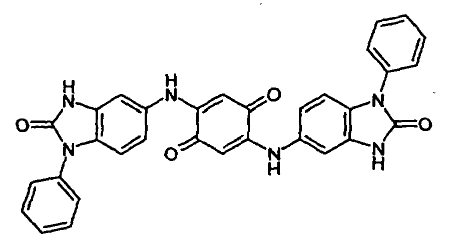
86 Teile dieses Zwischenproduktes werden unter Eiskühlung in 500 Teile H₂SO₄ (90%) eingetragen. Anschliessend werden innert 3 Stunden 29,6 Teile MnO₂ (88%) zugegeben und innert 24 Stunden auf Raumtemperatur erwärmt. Durch Zugabe von 63 Teilen Wasser wird das Kondensationsgemisch auf 80% verdünnt und unter Kühlung mit 5,1 Teilen H₂O₂ versetzt. Das erhaltene Pigment wird abfiltriert und mit 300 Teilen 80% H₂SO₄, danach mit 300 Teilen 50% H₂SO₄ und mit 4000 Teilen Wasser säurefrei gewaschen. Der feuchte Presskuchen wird dann in 500 Teilen Dimethylacetamid suspendiert und das verbleibende Wasser bei 100-150°C abdestilliert. Nach Kühlung auf 120°C wird das Pigment filtriert, dann mit 800 Teilen 120°C heissem Dimethylacetamid und danach mit 400 Teilen Wasser gewaschen und bei 80°C im Vakuum getrocknet. Es werden 60 Teile eines Pigments der obenstehenden Formel erhalten, das Kunststoffe in violetten Tönen mit ausgezeichneter Migrierechtheit färbt. 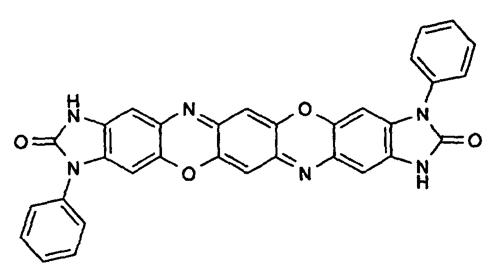
NMR ¹H (360 MHz, D₆-DMSO): 6.3 (s, 2H, aromatic CH), 6.6 (s, 2H, aromatic CH), 6.75 (s, 2H, aromatic CH), 7.3 (m, 2H, phenyl CH), 7.5 (m, 8H, phenyl CH),
Microanalysis. C 69.8 H 3.3 N 15.27 O 11.62 %; Found C 67.3 H 3.4 N 15.2 O 12.3 %.

### BEISPIEL 4

### 3,11-Di-(p-chloro-phenyl)-diimidazo(4,5-b:4',5-m)triphendioxazin-2,10-dion

In 400 Vol. Teile Eisessig werden 145 Teile 1-(p-chloro-phenyl)-1,3-Dihydro-5-aminobenzimidazol-2-on und 35 Teile 2,5-Dihydroxy-1,4-benzochinon eingetragen. Das Gemisch wird unter Rühren auf 110°C erhitzt, abgekühlt und das auskristallisierte Zwischenprodukt abfiltriert. Dieses wird mit 300 Teilen Eisessig, dann mit 500 Teilen Wasser gewaschen und getrocknet. 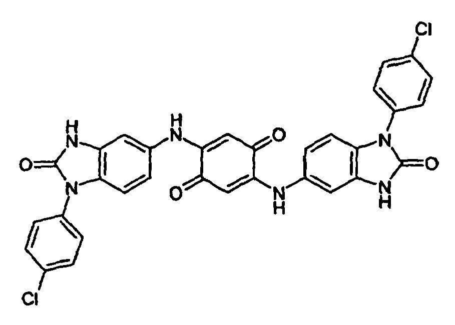
86 Teile dieses Zwischenproduktes werden unter Eiskühlung in 500 Teile H₂SO₄ (90%) eingetragen. Anschliessend werden innert 3 Stunden 29,6 Teile MnO₂ (88%) zugegeben und innert 24 Stunden auf Raumtemperatur erwärmt. Durch Zugabe von 63 Teilen Wasser wird das Kondensationsgemisch auf 80% verdünnt und unter Kühlung mit 5,1 Teilen H₂O₂ versetzt. Das erhaltene Pigment wird abfiltriert und mit 300 Teilen 80% H₂SO₄, danach mit 300 Teilen 50% H₂SO₄ und mit 4000 Teilen Wasser säurefrei gewaschen. Der feuchte Presskuchen wird dann in 500 Teilen Dimethylacetamid suspendiert und das verbleibende Wasser bei 100-150°C abdestilliert. Nach Kühlung auf 120°C wird das Pigment filtriert, dann mit 800 Teilen 120°C heissem Dimethylacetamid und danach mit 400 Teilen Wasser gewaschen und bei 80°C im Vakuum getrocknet. Es werden 60 Teile eines Pigments der obenstehenden Formel erhalten, das Kunststoffe in rotvioletten Tönen mit ausgezeichneter Migrierechtheit färbt. 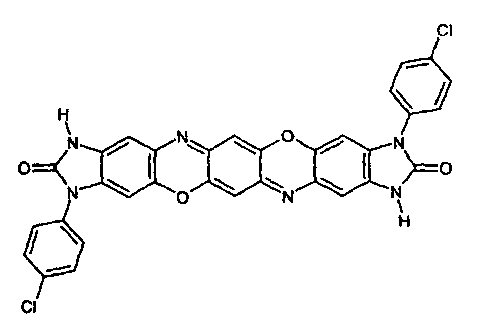
NMR ¹H (360 MHz, D₆-DMSO): 6.3 (s, 2H, aromatic CH), 6.6 (s, 2H, aromatic CH), 6.8 (s, 2H, aromatic CH), 7.5 (dd, 8H, phenyl CH). µA: calc.: C 62.05 H 2.6 N 13.57 %; found: C 61.9 H 2.9 N 13.3 %.

### BEISPIEL 5

### 3,11-Di-ethyl-diimidazo(4,5-b:4',5'-m)triphendioxazin-2,10-dion

In 400 Vol. Teilen Eisessig werden 75 Teile 1-Ethyl-5-aminobenzimidazol-2-on und 35 Teile 2,5-Dihydroxy-1,4-benzochinon eingetragen. Das Gemisch wird unter Rühren auf 110°C erhitzt, abgekühlt und das auskristallisierte Zwischenprodukt abfiltriert. Dieses wird mit 200 Teilen Eisessig, dann mit Wasser gewaschen und getrocknet.
Oder:

In 400 Vol. Teilen Eisessig werden 75 Teile 1-Ethyl-5-aminobenzimidazol-2-on und 45 Teile 2,5-Dimethoxy-1,4-benzochinon eingetragen. Das Gemisch wird unter Rühren auf 110°C erhitzt, abgekühlt und das auskristallisierte Zwischenprodukt abfiltriert. Dieses wird mit 200 Teilen Eisessig, dann mit Wasser gewaschen und getrocknet.
Oder:

In 400 Vol. Teilen 10% konzentrierter Salzsäure werden 75 Teile 1-ethyl-5-aminobenzimidazol-2-on eingetragen und anschliessend werden innert 2 Stunden 81 Teile 1,4-Benzochinon zugegeben. Das Gemisch wird unter Rühren auf 100°C erhitzt, abgekühlt und das auskristallisierte Zwischenprodukt abfiltriert. Dieses wird mit 800 Teilen warmer 10% konzentrierter Salzsäure, dann mit warmem Wasser gewaschen und getrocknet. 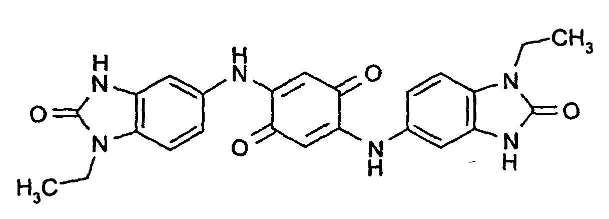
50 Teile dieses Zwischenproduktes werden unter Eiskühlung in 300 Teile H₂SO₄ (95%) eingetragen. Anschliessend werden innert 3 Stunden 13 Teile MnO₂ zugegeben und innert 24 Stunden auf Raumtemperatur erwärmt. Durch Zugabe von 94 Teilen Wasser wird das Kondensationsgemisch auf 80% verdünnt und unter Kühlung mit 3,6 Teilen H₂O₂ versetzt. Das erhaltene Pigment wird abfiltriert und mit 600 Teilen 80% H₂SO₄, danach mit 300 Teilen 50% H₂SO₄ und mit 4000 Teilen Wasser säurefrei gewaschen. Der feuchte Presskuchen wird dann in 500 Teilen Dimethylacetamid suspendiert und das verbleibende Wasser bei 100-150°C abdestilliert. Nach Kühlung auf 120°C wird das Pigment filtriert, dann mit 800 Teilen 120°C heissem Dimethylacetamid und danach mit 400 Teilen Wasser gewaschen und bei 80°C im Vakuum getrocknet. 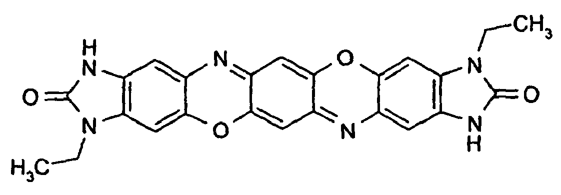
NMR ¹H (360 MHz, D₆-DMSO): 1.11 (t, 6H, CH₃), 3.71 (q, 4H, CH₂), 6.25 (s, 2H, aromatic CH), 6.5 (s, 2H, aromatic CH), 6.6 (s, 2H, aromatic CH).
Micronanalysis. C 63.4 H 4.0 N 18.5 O 14.1 %; Found C 62.5 H 3.7 N 18.8 O 14.1 %.

Das erhaltene Pigment der obenstehenden Formel färbt Kunststoffe in rötlich-blauen Tönen mit ausgezeichneter Migrier- und Lösungsmittelechtheit.

## Patentansprüche

1. Verfahren zur Herstellung von symmetrisch disubstituierten Triphendioxazin-Verbindungen, **dadurch gekennzeichnet, dass** eine Verbindung der Formel (IV) 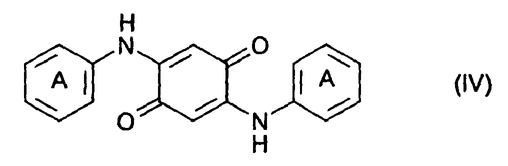
worin die mit A bezeichneten Kerne linear in 3,4-Stellung anelliert Ringe mit den Restgliedern der Formeln -NR₁(CO)ₘ-NR₂ oder -O-CO-NR₂ enthalten; m 1 oder 2 ist; und R₁ bzw. R₂ unabhängig voneinander, Wasserstoff, einen C₁₋₈Alkylrest, einen substituierten oder unsubstituierten Phenyl-, Benzyl-, Naphthyl- oder Benzanilid-Rest, einen substituierten oder unsubstituierten C₅₋₆Cycloalkyl-Rest oder einen Rest der Formel 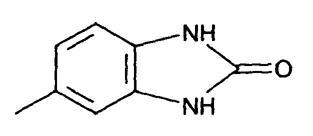 bedeuten,
in Gegenwart von Braunstein (Mangandioxid) und 80 bis 100%iger Schwefelsäure cyclisiert wird.

2. Verfahren zur Herstellung von Verbindungen der Formel (IV) 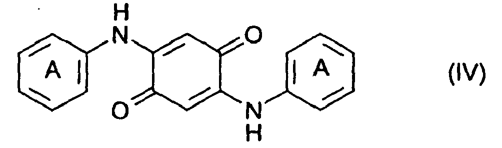
**dadurch gekennzeichnet, dass** ein Mol der Verbindung der Formel (II) 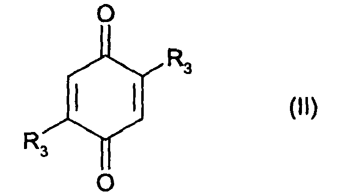
worin R₃ Wasserstoff, einen C₁₋₁₀Alkoxy, Halogen oder Hydroxy bedeutet, mit zwei Mol einer Verbindung der allgemeinen Formeln (III), 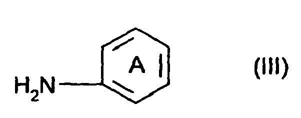
worin A entsprechend Anspruch 1 definiert ist, kondensiert.

3. Verfahren nach Anspruch 2, **dadurch gekennzeichnet dass** ein Mol der Verbindung der Formel (II) mit 2 Mol einer Verbindung der allgemeinen Formeln (IIIa) bis (IIId) 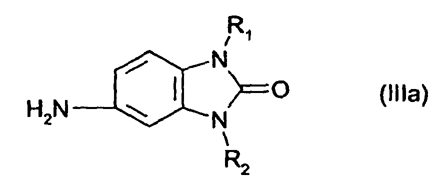 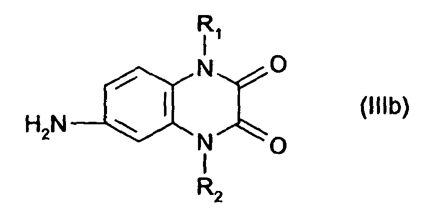 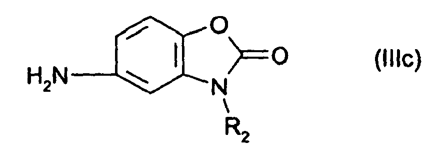 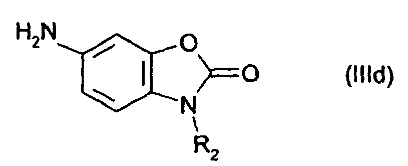
worin R₁ und R₂ wie in Anspruch 1 definiert sind, kondensiert.

4. Verfahren nach Anspruch 2 oder 3, **dadurch gekennzeichnet, dass** R₃ eine Hydroxygruppe ist.

5. Verfahren nach Anspruch 2, 3 oder 4, **dadurch gekennzeichnet, dass** die Kondensation in Gegenwart einer anorganischen oder organischen Säure bei Temperaturen zwischen 50 und 160°C erfolgt.

6. Verbindungen der allgemeinen Formel (IV) 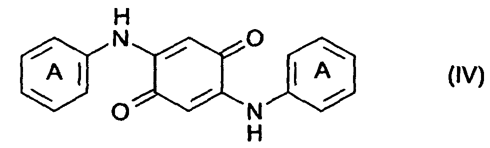
worin die mit A bezeichneten Kerne linear in 3,4-Stellung anelliert Ringe mit den Restgliedern der Formeln -NR₁(CO)ₘ-NR₂ oder -O-CO-NR₂ enthalten; m 1 oder 2 ist; und R₁ bzw. R₂ unabhängig voneinander, Wasserstoff, einen C₁₋₈Alkylrest, einen substituierten oder unsubstituierten Phenyl-, Benzyl-, Naphthyl- oder Benzanilid-Rest, einen substituierten oder unsubstituierten C₅₋₆Cycloalkyl-Rest oder einen Rest der Formel 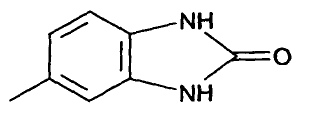
bedeuten.

7. Verbindungen der Formel (IV) nach Anspruch 6 **gekennzeichnet dadurch, dass** R₁ und R₂ unabhängig voneinander, Wasserstoff, einen Methyl-Rest, einen Ethyl-Rest, einen (n,i) Propyl-Rest, einen (n, i, sek., tert.) Butyl-Rest, einen Cyclohexyl-Rest, einen substituierten oder unsubstituierten Benzanilid-Rest, einen Naphthyl-Rest, einen Rest der Formel 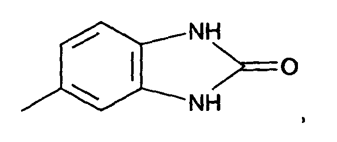
einen unsubstituierten Phenyl-Rest, einen Phenyl-Rest, der einfach oder mehrfach substituiert ist durch Reste aus der Gruppe Halogen, vorzugsweise Chlor, Nitro, Phenyl, C₁₋₈-Alkyl, vorzugsweise C₁₋₄-Alkyl, und C₁₋₂-Alkoxy, bedeuten.

8. Verbindungen der Formel (IV) nach Anspruch 7 **gekennzeichnet dadurch, dass** der substituierte Phenylrest in R₁ und R₂ ein Rest der Formeln (a) bis (q), 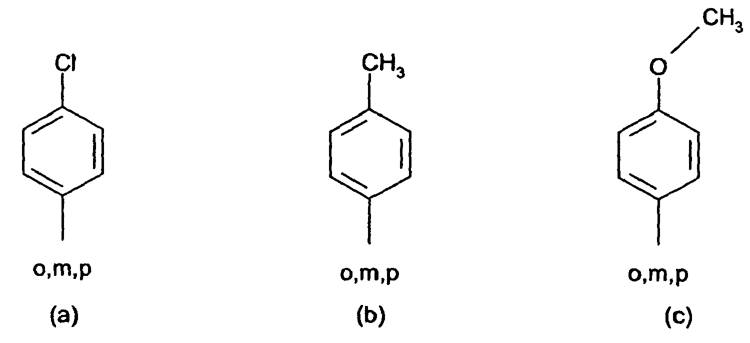 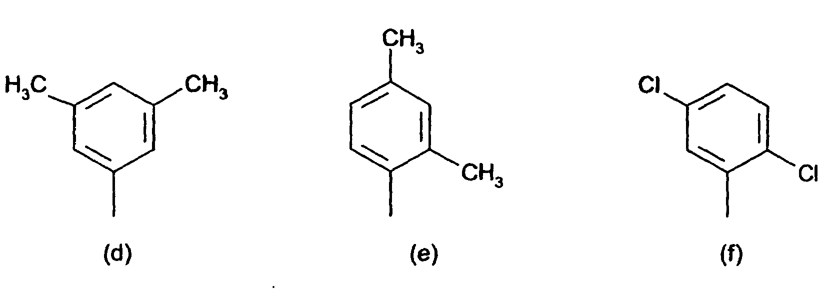 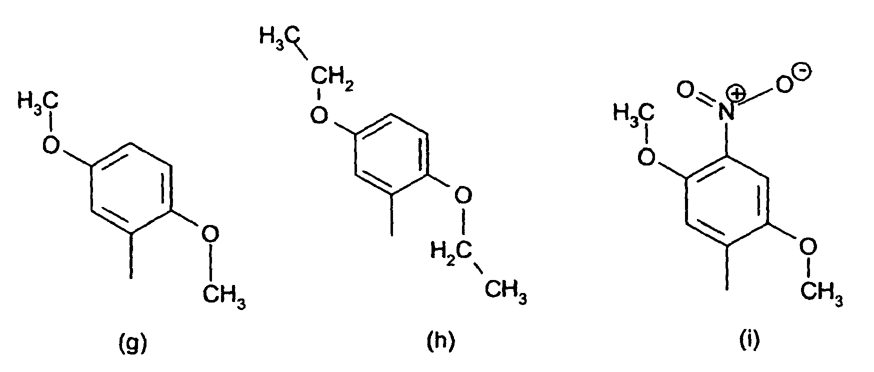 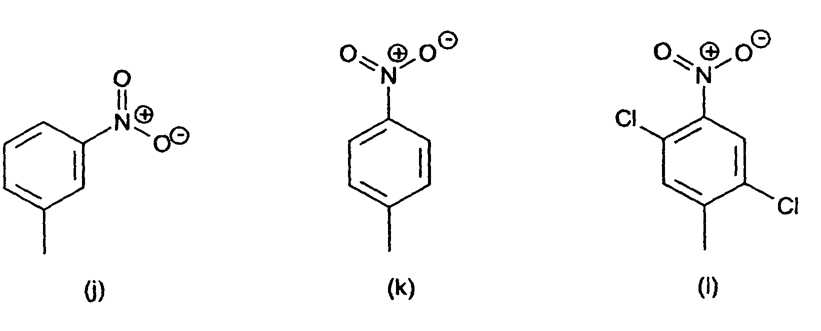 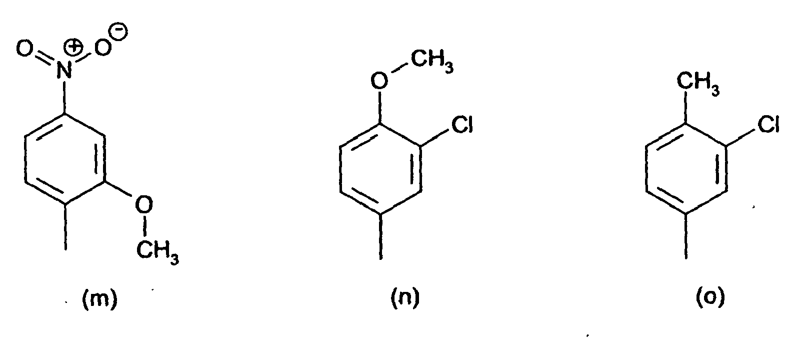 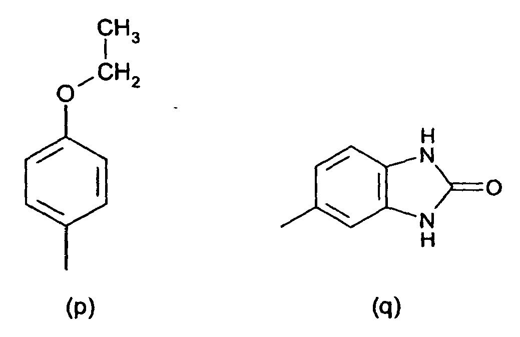
bzw. der substituierte Benzanilid-Rest in R₁ und R₂ ein Rest der Formeln (r) und (s) ist.

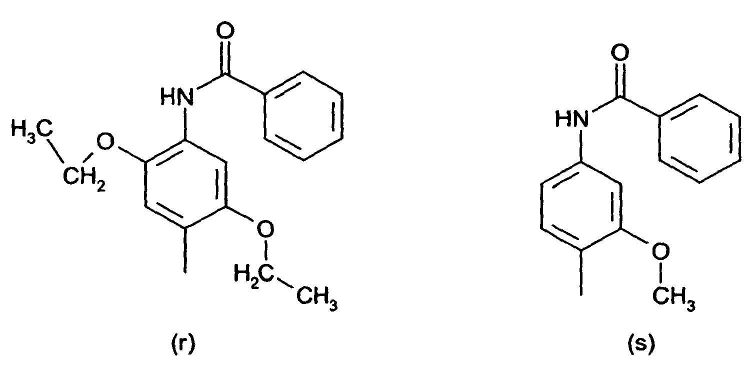

## Claims

1. A process for preparing symmetrically disubstituted triphendioxazine compounds, which comprises cyclizing a compound of the formula (IV) 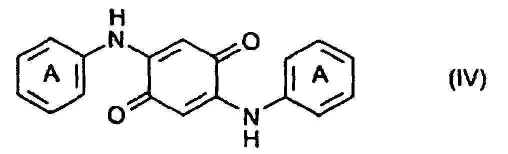
where the rings marked A are each linearly fused in position 3, 4 with a ring having a complementary member of the formula -NR₁(CO)ₘ-NR₂ or -O-CO-NR₂; m is 1 or 2; and R₁ and/or R₂ are each independently hydrogen, a C₁₋₈alkyl radical, a substituted or unsubstituted phenyl, benzyl, naphthyl or benzanilide radical, a substituted or unsubstituted C₅₋₆cycloalkyl radical or a radical of the formula 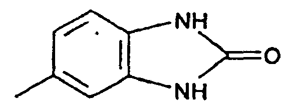
in the presence of manganese dioxide and 80 to 100% strength sulfuric acid.

2. A process for preparing a compound of the formula (IV) 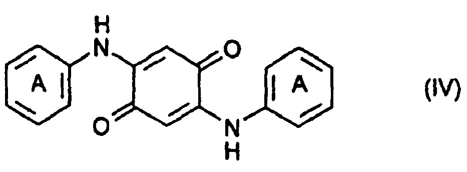
which comprises condensing one mole of the compound of the formula (II) 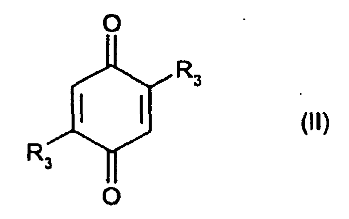
where R₃ s hydrogen, C₁₋₁₀alkoxy, halogen or hydroxyl, with two moles of a compound of the general formula (III) 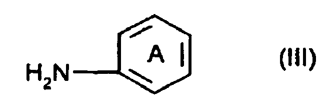
where A is as defined in claim 1.

3. A process according to claim 2, wherein one mole of the compound of the formula (II) is condensed with 2 mol of a compound of general formulae (IIIa) to (IIId) 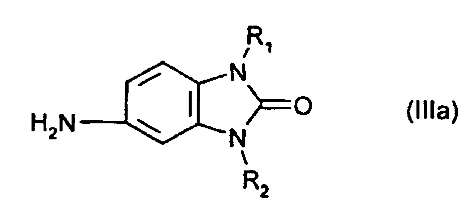 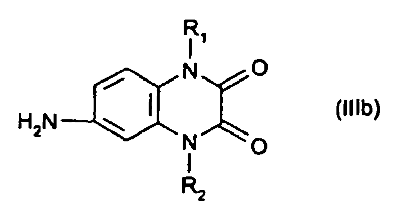 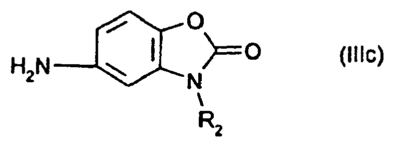 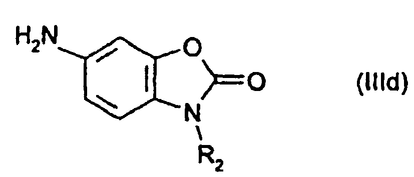
where R₁ and R₂ are each as defined in claim 1.

4. A process according to claim 2 or 3, wherein R₃ is hydroxyl.

5. A process according to claim 2, 3 or 4, wherein said condensing is effected in the presence of an inorganic or organic acid at temperatures between 50 and 160°C.

6. A compound of the general formula (IV) 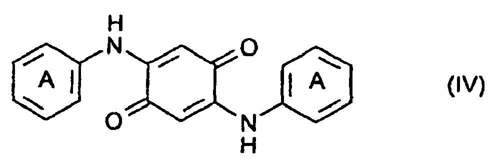
where the rings marked A are each linearly fused in position 3, 4 with a ring having a complementary member of the fonnula -NR₁(CO)ₘ-NR₂ or -O-CO-NR₂; m is 1 or 2; and R₁ and/or R₂ are each independently hydrogen, a C₁₋₈alkyl radical, a substituted or unsubstituted phenyl, benzyl, naphthyl or benzanilide radical, a substituted or unsubstituted C₅₋₆cycloalkyl radical or a radical of the formula

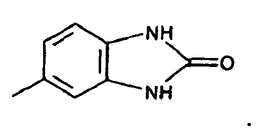

7. A compound according to claim 6 of the formula (IV), wherein R₁ and R₂ are each independently hydrogen, methyl, ethyl, n-propyl, isopropyl, n-butyl, isobutyl, sec-butyl, tert-butyl, cyclohexyl, a substituted or unsubstituted benzanilide radical, naphthyl, a radical of the formula 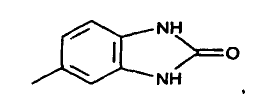
an unsubstituted phenyl radical or a phenyl radical which is substituted by one or more substituents selected from the group consisting of halogen, preferably chlorine, nitro, phenyl, C₁₋₈-alkyl, preferably C₁₋₄-alkyl, and C₁₋₂₋alkoxy.

8. A compound according to claim 7 of the formula (IV), wherein the substituted phenyl radical in R₁ and R₂ is a radical of the formulae (a) to (q) 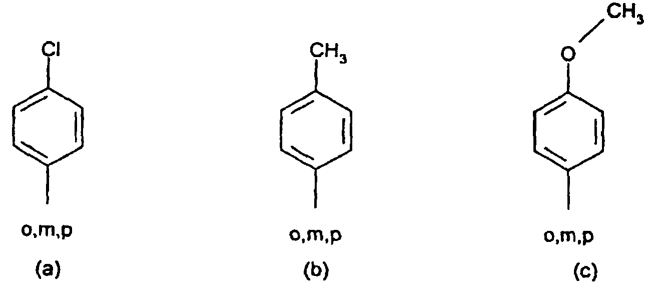 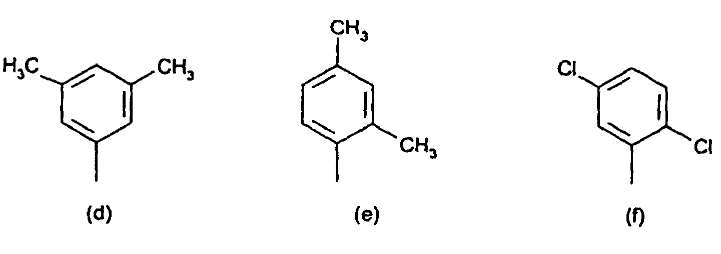 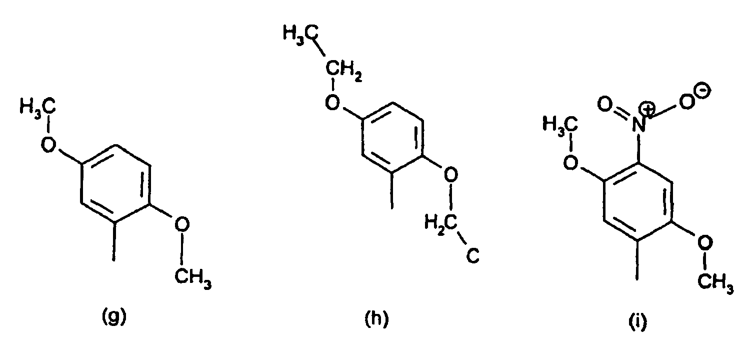 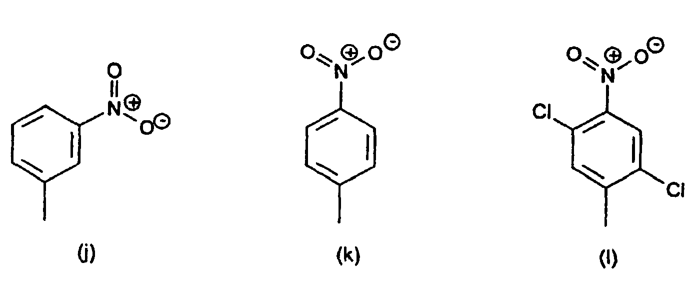 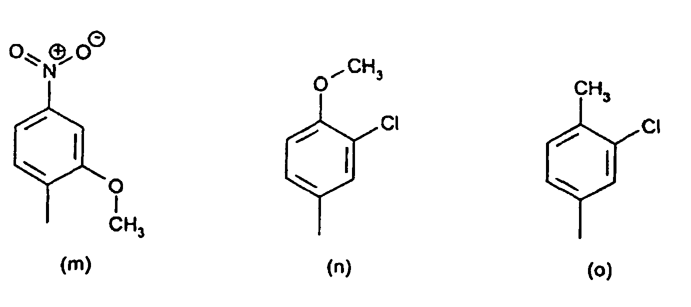 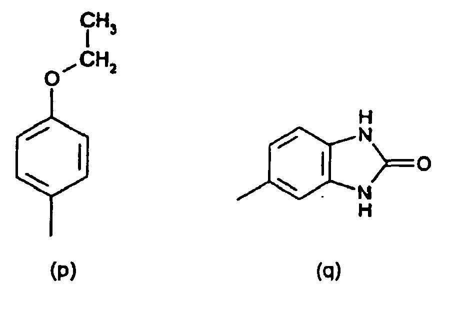
or the substituted benzanilide radical R₁ and R₂ is a radical of the formulae (r) and (s)

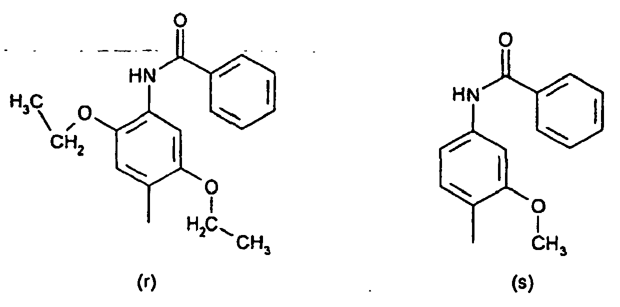

## Revendications

1. Procédé de préparation de triphènedioxazines à disubstitution symétrique, **caractérisé en ce qu'**un composé de formule (IV) 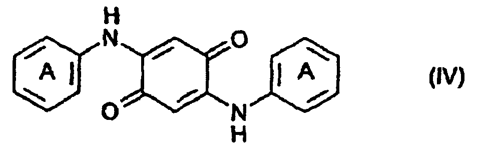
dans laquelle les noyaux représentés par A contiennent, d'une manière linéaire, des noyaux condensés en position 3,4, avec des termes résiduels ayant les formules -NR₁(CO)ₘ-NR₂ ou -O-CO-NR₂, m vaut 1 ou 2 ; et R₁ ou R₂ représentent chacun indépendamment de l'autre un atome d'hydrogène, un radical alkyle en C₁₋₈, un radical phényle, benzyle, naphtyle ou benzanilide substitué ou non substitué, un radical cycloalkyle en C₅₋₆ substitué ou non substitué, ou un radical de formule 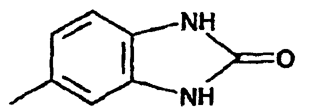 est soumis à une cyclisation en présence de pyrolusite (dioxyde de manganèse) et d'acide sulfurique à 80 à 100 %.

2. Procédé de préparation de composés de formule (IV) 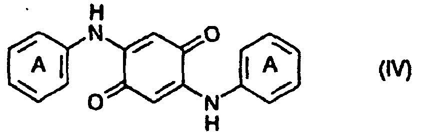
**caractérisé en ce qu'**on condense une mole du composé de formule (II) 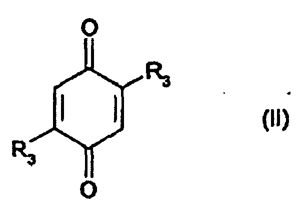
dans laquelle R₃ est un atome d'hydrogène, un groupe alcoxy en C₁₋₁₀, halogéno ou hydroxy, avec deux moles d'un composé de formule générale (III) 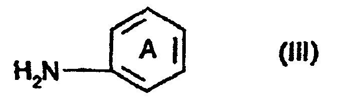
dans laquelle A est défini conformément à la revendication 1.

3. Procédé selon la revendication 2, **caractérisé en ce qu'**on condense une mole du composé de formule (II) avec 2 moles d'un composé de formule générale (IIIa) à (IIId) 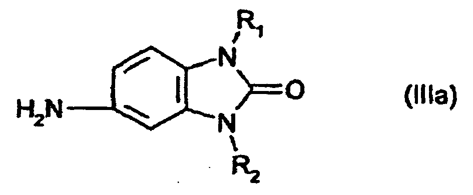 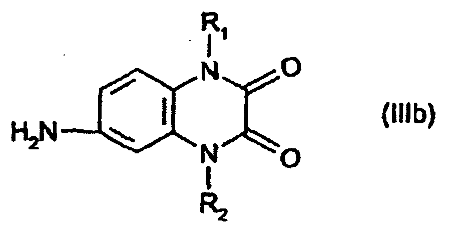 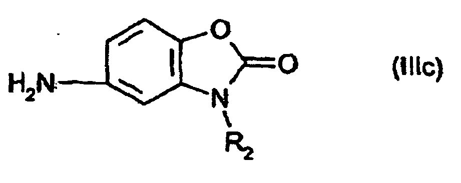 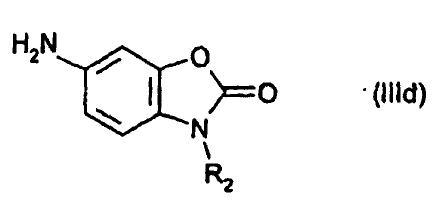
dans lesquelles R₁ et R₂ sont tels que définis dans la revendication 1.

4. Procédé selon la revendication 2 ou 3, **caractérisé en ce que** R₃ est un groupe hydroxy.

5. Procédé selon la revendication 2, 3 ou 4, **caractérisé en ce que** la condensation est mise en oeuvre en présence d'un acide organique ou inorganique à des températures de 50 à 160°C.

6. Composés de formule générale (IV) 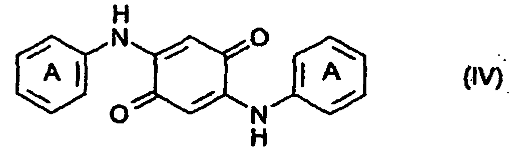 dans laquelle les noyaux représentés par A contiennent, d'une manière linéaire, des noyaux condensés en position 3,4, avec des termes résiduels ayant les formules -NR₁(CO)ₘ-NR₂ ou -O-CO-NR₂, m vaut 1 ou 2 ; et R₁ ou R₂ représentent chacun indépendamment de l'autre un atome d'hydrogène, un radical alkyle en C₁₋₈, un radical phényle, benzyle, naphtyle ou benzanilide substitué ou non substitué, un radical cycloalkyle en C₅₋₆ substitué ou non substitué, ou un radical de formule

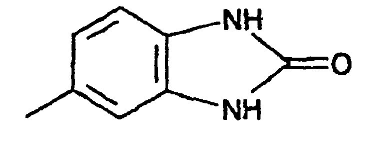

7. Composés de formule (IV) selon la revendication 6, **caractérisés en ce que** R₁ et R₂ représentent chacun indépendamment de l'autre un atome d'hydrogène, un groupe méthyle, un groupe éthyle, un groupe (n, iso)-propyle, un groupe (n, iso, sec, tert)-butyle, un groupe cyclohexyle, un groupe benzanilide substitué ou non substitué, un groupe naphtyle, un radical de formule 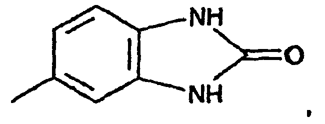 un radical phényle non substitué, un radical phényle, qui est une ou plusieurs fois substitué par des substituants choisis dans l'ensemble comprenant les groupes halogéno, de préférence chloro, nitro, phényle, alkyle en C₁₋₈, de préférence alkyle en C₁₋₄, et alcoxy en C₁₋₂.

8. Composés de formule (IV) selon la revendication 7, **caractérisés en ce que** le radical phényle substitué de R₁ et R₂ est un radical ayant les formules (a) à (q) 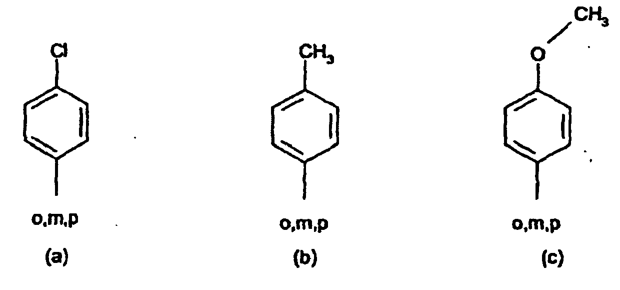 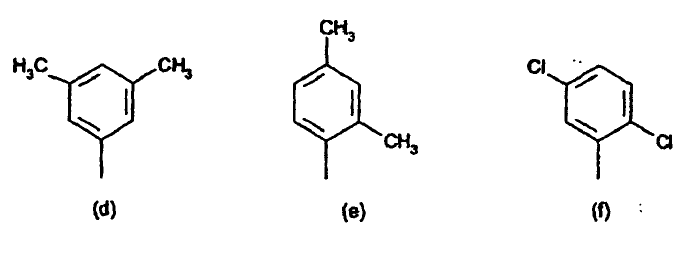 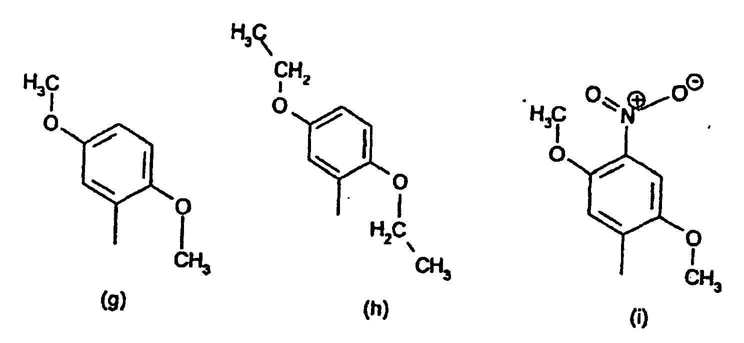 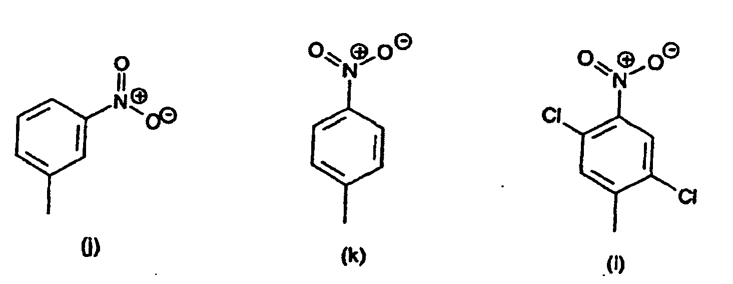 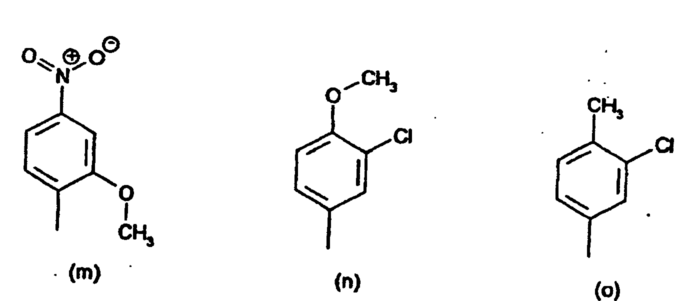 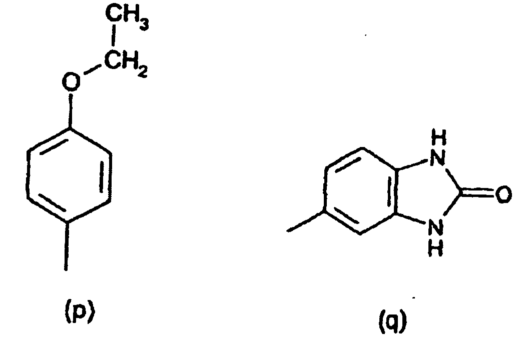
ou encore le radical benzanilide substitué de R₁ et R₂ est un radical ayant les formules (r) et (s) :
